Europäisches Patentamt

European Patent Office

Office européen des brevets

(11) Publication number: **0 172 873**
**B1**

(12) **EUROPEAN PATENT SPECIFICATION**

(45) Date of publication of patent specification: **10.05.89**

(21) Application number: **85901111.6**

(22) Date of filing: **12.02.85**

(86) International application number:
**PCT/SE85/00070**

(87) International publication number:
**WO 85/03441 15.08.85 Gazette 85/18**

(51) Int. Cl.⁴: **A 61 K 35/48, A 61 K 35/55, A 61 K 37/22, C 07 G 17/00**

(54) AN ERECTION AND FERTILITY PROMOTING AGENT, A METHOD FOR PRODUCING THE SAME, AND THE USE THEREOF IN THE PRODUCTION OF ERECTION AND FERTILITY PROMOTING COMPOSITIONS.

(30) Priority: **13.02.84 SE 8400752**

(43) Date of publication of application:
**05.03.86 Bulletin 86/10**

(45) Publication of the grant of the patent:
**10.05.89 Bulletin 89/19**

(84) Designated Contracting States:
**AT BE CH DE FR GB LI LU NL SE**

(56) References cited:
**DE-C- 670 599**
**SE-B- 309 648**

"Patent Abstracts of Japan, abstract of JP 58-29711 (A), publ. 1983-02-22".
Journal of Endocrinology, vol. 58, no. 2, issued 1973 (London), M. RAJALAKSHMI et al.
"Distribution of fructose, citric acid, sialic acid and lipids in male accessory glands of the hamster, Mesocricetus auratus", pp. 349-350
Biochemical Pharmacology, vol. 31, no. 21, issued 1982 (Oxford), B.V.R. SASTRY et al.

(73) Proprietor: **FERRING, B.V.**
**Waarderweg 45 P.O. Box 553**
**NL-2003 RN Haarlem (NL)**

(72) Inventor: **Hellgren, Lars Gustav Inge**
**Bronsgjutaregatan 13**
**S-421 63 Västar Frölunda (SE)**

Inventor: **Mylius, Erling A.**
**Ragnhildsgatan 11**
**N-7000 Trondheim (NO)**

Inventor: **Vincent, Jan Gustav**
**Linnégatan 31**
**S-113 47 Stockholm (SE)**

(74) Representative: **Nilsson, Brita et al**
**AWAPATENT AB Box 5117**
**S-200 71 Malmö (SE)**

(56) References cited:

"Enkephalin - and substance P-like immunoreactivities of mammalian sperm and accessory sex glands", pp. 3519-3522
Biology of Reproduction, vol. 3, no. 3, issued 1970 (New York), R.G. HART, "Cowper's gland secretion in rat semen coagulation. I. Isolation and amino acid analysis of the seminal vesicle substrate", pp. 347-352

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 172 873 B1

## Description

The invention relates to an agent isolated from Cowper's glands of human males and male mammals and corresponding organs; the large vestibularis glands, Bartholini's glands of women and female mammals, hereinafter called the glands; a method for producing said agent; and medicaments or veterinary medicinal compositions containing this agent as the active component.

Lipid extracts from the glands have been found to possess properties which lead one to expect, on good grounds, that they will promote erection, ejaculation, mobility of the spermatozoa and the ability thereof to penetrate protein and the female reproduction organs. The agent can therefore constitute an active component in pharmaceutical preparations with this indication.

The glands of human and male mammals are named Cowper's glands (also bulbo-urethral glands) and consist of paired symmetrical composite alveola glands of said consistency, greyish yellow or slightly brown in colour and of the size of a common garden pea or kidney beam. Their excretion duct is up to 4 cm in length and opens into the urethra immediately adjacent the location at which the urethra opens into the corpus cavernosum penis.

The homologous glands of the female human and female mammal is called the Bartholini's gland and consists of two greyish yellow, round or oval glands having the size of a common garden peak or kidney bean and lying in the dorsal side of the vagina orifice. The excretion duct is from 1.5 to 2 cm in length and opens into introitus vagina. They are the direct correspondence to the Cowper's glands of the male species. The function of the glands is not fully understood. With regard to the Cowper's glands of male animals, it is known that the total extract of the secretion from the glands coagulates the semen. In this regard there is formed a mucous clot which prevents reflux of seminal fluid upon ejaculation. R. G. Hart, The mechanism of Cowper's secretion in caogulating cat semen. J. Reprod. Fert. (1968), 17, 223—226. J. J. Gueze and J. W. Slot Synthesis and secretion of glycoproteins in rat bulbo-urethral (Cowper's) glands, Biology of reproduction 15, 118—125 (1976).

The biological effect of the lipid fractions deriving from the glands has not previously been investigated. Consequently, the present discovery of the potency effect of the lipid fractions on various parts of the urogenital sphere is of great importance in determining the function of the glands. Lipid fractions derived from these glands, taken from males and females of the human species, bulls, boards, rams, sheep and cows have been investigated and when subjected to pharmacological tests, found to have approximately the same properties as those set forth below.

The inventors have thus now surprisingly found in the lipid fraction from glands deriving from the above-named species a factor which appears to influence both erection and ejaculation as well as the mobility of the spermatozoa and its ability to penetrate protein in humans and animals. The factor would seem to be a potent peptide lipid mediator of prostaglandin-like/leucotriene-like type.

The invention relates to an agent consisting of such lipid fractions taken from the glands of humans and animals as those that contract gerbil colon. Prostaglandins and substances similar thereto contract gerbil colon, and consequently the contraction of gerbil colon is used as a criterium of the biological activity of such substances. The test associated herewith is conducted as follows: The upper part of a colon removed from a gerbil is suspended in a conventional intestine bath, the lower attachment being fixed while the upper attachment is connected to a sensor which registers contraction and relaxation of the intestine under the influence of various substances.

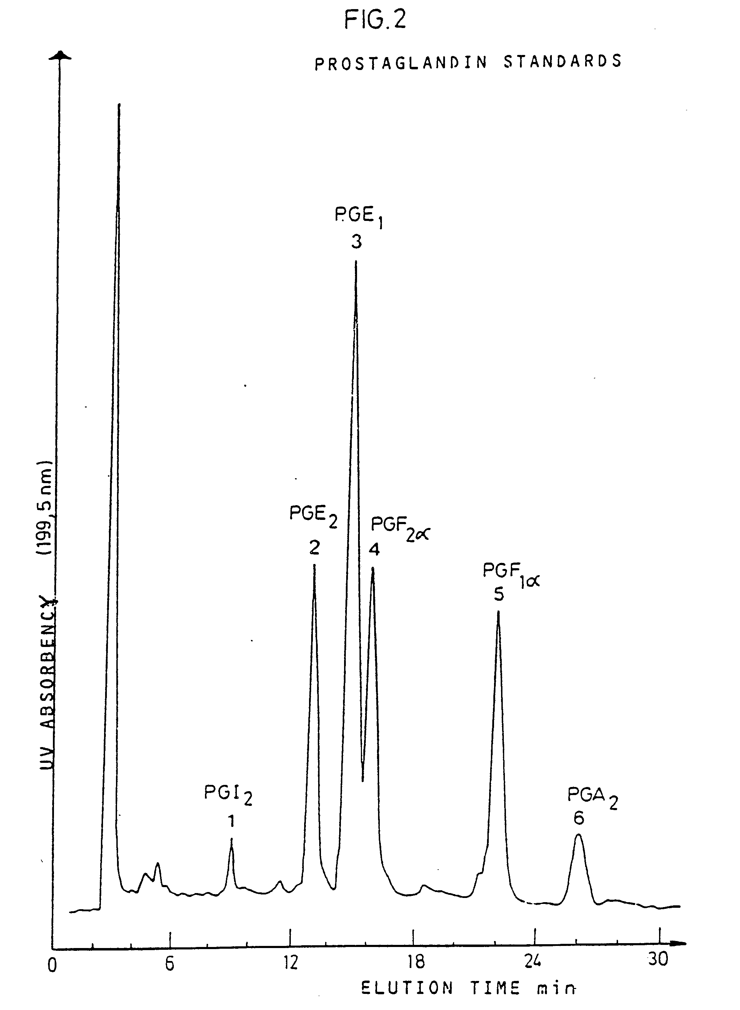

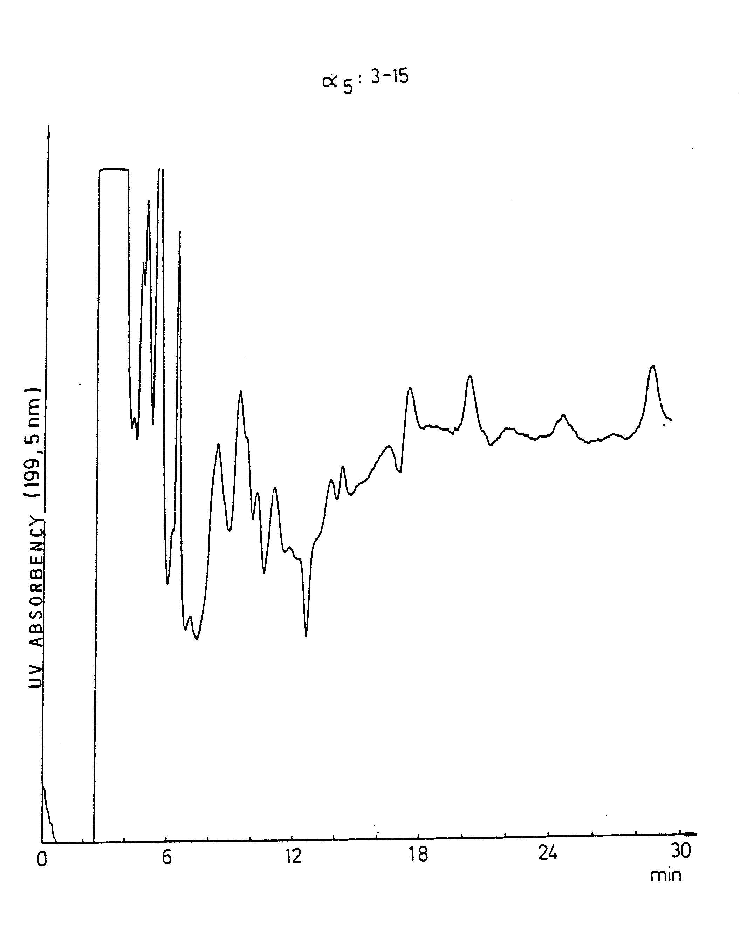

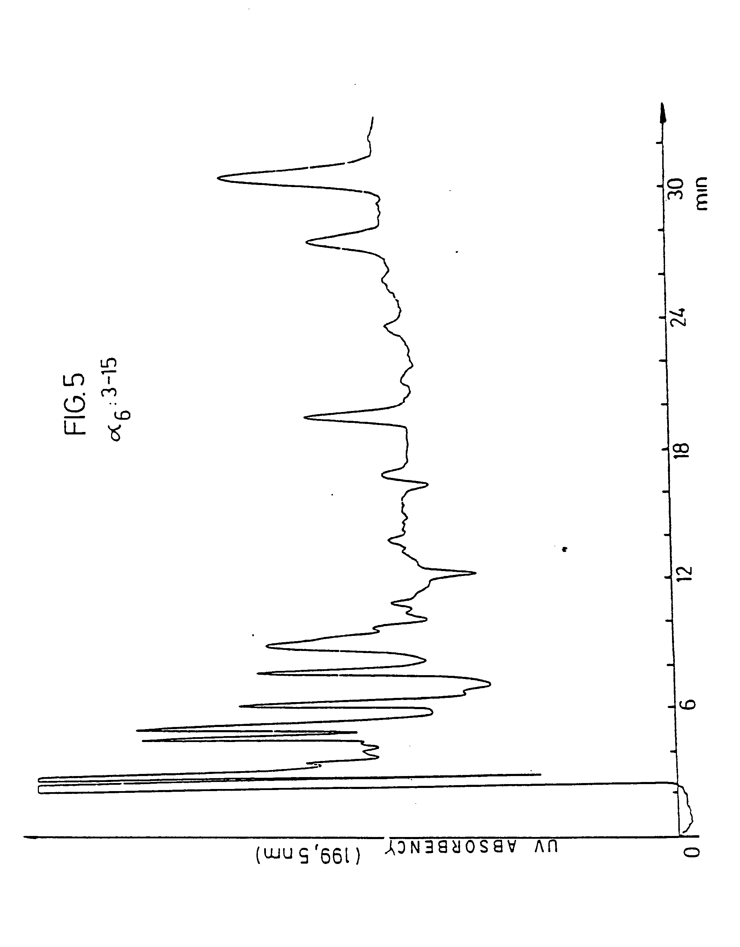

Since the agents contracts gerbil colon, there are good grounds to suppose that it contains prostaglandin-like substances. However, no known prostaglandins have been shown to be present in those lipid fractions taken from the glands which contract gerbil colon and which are the agent according to the invention (c.f. the Example and Figures 2, 3, 4 and 5, from which it will be seen that it has not been possible to show the prostaglandins $PGI_2$, $PGE_2$, $PGE_2$, $PGF_{2\alpha}$, $PGA_2$ in such lipid fractions from the glands as those contracting gerbil colon when applying known techniques). Consequently, the contractions of gerbil colon can be used as a criterium that the factor possessing fertility promoting properties according to the invention is present in the lipid extract from the glands.

The factor reacts positively to ninhydrin, which indicates that it contains amino groups. Tests employing dispersive X-ray analysis (KEVEX) show the presence of sulphur (S) in the factor. These two circumstances—the positive reaction to ninhydrin and the presence of sulphur—indicates that similarities exist between the factor and the leucotrienes. Leucotrienes, however, do not contract gerbil colon. On the other hand, they do contract potent uterus musculature, as does also the agent.

Gas chromatographic-mass spectrometric analysis shows a complex mixture where, however, certain fragments indicate the presence of neuro transmitter-like substances having a molecular weight between 200—1000 m/e.

NMR (nuclear magnetic resonance) of a fraction of high biological activity as exhibited all the characteristics of prostaglandin-like substances, such as: long molecular chain, the presence of fatty acids, a terminal $CH_3$-group.

The agent according to the invention has been prepared by methods conventional in the extraction and separation of prostaglandins. Thus, the glands can first be freed from proteins i.e. ethanol extraction and the residue then extracted at an acid pH with diethyl ether, which dissolves lipids. The glands can also be extracted directly in an acid environment, one or more times, with a solvent, and in particular with a lipid solvent such as ethyl acetate, isopropanol, methanol, acetone, chloroform, and mixtures thereof such as chloroform/methanol 2:1, vol/vol; ethyl acetate in mixture with isopropanol, methanol, acetone in varying percent by volume with water or the lipid solvents mentioned above, preferably methanol or ethyl acetate/methanol 3:1, vol/vol, and the pH adjusted with a buffer to pH 3.5—5, preferably to 4.5, with 0.1 M KH-phthalate. The water is then removed in a conventional manner, e.g. by freeze drying. The extract may also be allowed to stand overnight in the freezer at a temperature beneath 0°C and the formed ice crystals filtered off at low temperatures, beneath 0°C. The work is suitably effected in a nitrogen environment, to shield against oxidative effects from atmospheric oxygen. The sample is then optionally vapourized in a water bath (40°C), preferably in a nitrogen gas atmosphere. This sample can be used as the erection promoting substance according to the invention. The sample is optionally dissolved in a few millilitres of the lipid solvent mentioned above, preferably in ethyl acetate, and stored in a nitrogen environment at −20°C.

The sample can then be processed in accordance with known methods conventional in the separation of prostaglandins and those fractions which contract gerbil colon recovered. The separation can be effected by chromatography, on silica gel for example, preferably so-called dry-column chromatography while eluting with mixtures of ethyl acetate, isooctane, ethanol, water, acetic acid, chloroform, isopropanol and formic acid, preferably ethylacetate-isooctane-ethanol-acetic acid-water (in ratios of 35:10:3:0.1:0.1) or with chloroform-isopropanol-ethanol-formic acid (45:5:0.5:0.3) vol/vol. The respective fractions can then be eluted out with mixtures of chloroform and methanol, preferably in a volumetric ratio of 1:1.

The fractions may also be separated in silica-gel plates (TLC) or by column chromatography with the same or modified solvent system. Those fractions which contract gerbil colon are recovered and stored at −20°C for further analysis. These fractions can be used as the agent according to the invention, preferably after evaporating to dryness. They can also be further cleansed by chromatography, preferably after first being evaporated to dryness. The fractions can then be purified by chromatography, preferably in silica gel, e.g. by high-pressure—liquid chromatography while eluting with mixtures of phosphoric acid, acetone nitrile, methanol, preferably phosphoric acid—acetonitrile-methanol in ratios of 40:12:48.

The invention will now be described in more detail with reference to the accompanying drawings, in which

Fig. 1 illustrates a preferred method of producing the agent according to Example I;

Fig. 2 illustrates the U.V.-adsorption as a function of the elution volume with high pressure liquid chromatography of prostaglandin standards;

Figs. 3—5 illustrate the U.V.-adsorption as a function of the elution volume with high pressure liquid chromatography of the fractions $\alpha_4$, $\alpha_5$ and $\alpha_6$ respectively obtained from the chromatography on Silica Noelon® DCC in Example 1.

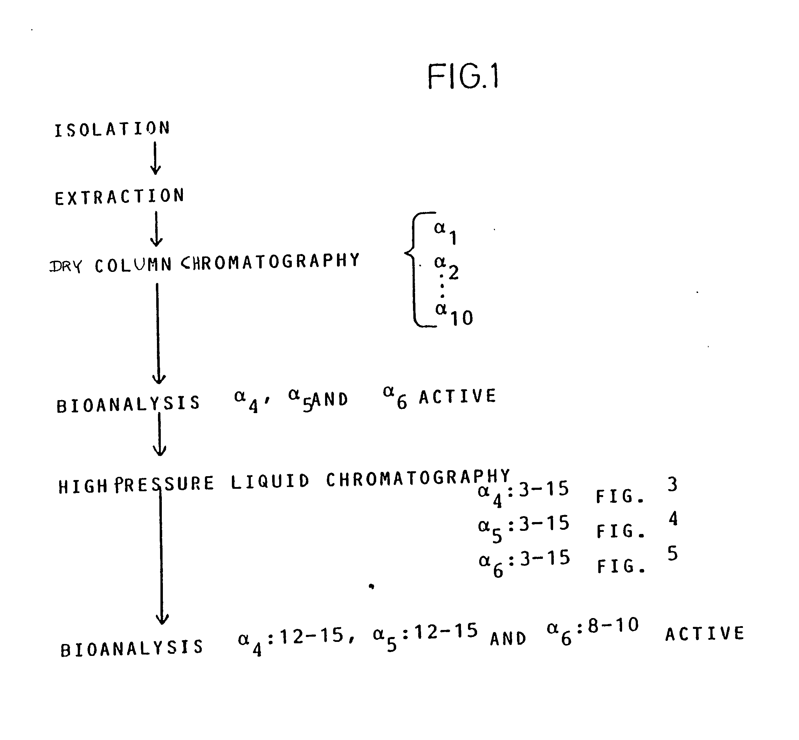

Examples of a preferred method of manufacture according to the invention are given in the following (c.f. also the review of Fig. 1).

Example 1

Isolation

The factor is isolated from the glands in the following manner: Cowper's glands are removed from fertile males who have newly died. The preparation is then immediately frozen down to a temperature of −20°C. Bartholini's glands are isolated from fertile females newly deceased and the preparations produced immediately frozen down to −20°C.

Extraction

Lipid extract was taken from the glands of Example 1 in accordance with the following process: 23.5 g of Cowper's glands and 16.1 g of Bartholini's glands were finely divided and homogenized each per se with an Ultraturrax® in 60 ml and 20 ml respectively of ethylacetate/methanol 3:1 vol/vol. The pH was adjusted with 0.1 M potassium hydrogen phthalate to 4.5.

The lipid extract was allowed to stand overnight in a freezer, and water crystals thereafter filtered off at a temperature below 0°C. The sample was then concentrated by evaporation in a water bath (40°C) in a nitrogen environment. Subsequent hereto there was obtained 0.43 g from Cowper's glands and 0.15 g from Bartholini's glands. The sample was then stored at a temperature of −20°C in a nitrogen gas atmosphere.

Separation and purification

The biological factor was separated with the aid of a modified method for dry column chromatography which successfully distinguishes the bioactive lipids from the remaining substances in the extract. There was used a column comprising 10 (ten) Teflon® rings, having a height of 2 cm and an inner diameter of 2.5 cm, tightly packed in a tube. Each ring corresponded to a fraction which extended between two sequential decimal (Rf−)-values. The column was filled with activated Silica Woelm® DCC, under vibration. Each of the aforesaid 0.43 g and 0.15 samples were introduced into one such column per se and eluted in the solvent mixture chloroform/isopropanol/ethanol/formic acid at ratios of 45:5:0.5:0.3 vol/vol.

The supply of solvent was held constant with the aid of the outlet of a separating funnel placed 1—2 cm above the sample. The position of the solvent front adjacent the bottom of the column was indicated with a first acoustic warning signal produced by an electric sensor. The final point was indicated by a second signal. A screw cap uppermost on the tube was then removed, so that the inner divided Teflon column could be pushed axially upwards with the aid of a plunger. The Teflon rings were readily separated from one another, one at a time, with rapid cutting movements. Finally, each ring was mounted in ten specially arranged Teflon columns for simultaneous elution. The resultant fractions were eluted with some milliliters of chloroform/methanol 1:1 vol/vol and the biological activity was tested on gerbil colon. The fractions 4, 5 and 6—the rings 4, 5 and 6 counted from the top are hereinafter referenced alpha-4, alpha-5, and alpha-6—were found to contract gerbil colon and possessed the properties shown in experiments 1—7. This applied to both the aforesaid fractions, from Cowper's and Bartholini's glands. The fractions were vaporized to dryness within a nitrogen gas environment in a water bath (40°C) and cleansed according to the following, although this cleansing step is optional.

High pressure—liquid chromatography (HPLC)-analysis

The fractions alpha-4, alpha-5 and alpha-6 isolated from respective glands were analysed by high pressure liquid chromatography, referred to as HPLC. Separation was effected on a high pressure 5 μm reversed-phase-column. A system comprising a Consta Metric®-111-HPLC-pump (Lab. Data Control) and a Consta Metric Spectromonitor® III (LDC) variable wave-length detector was used. The detector was coupled to an Rec-2-recorder® (Pharmacia) and an integrator (HP 3888 A). The column system comprised a 49×4.6 mm LD pre-column packed with 40 μm particulate reverse phase material (Pelliguar® LC-18) and a 250×4.6 LD, Supelcosil® LC-18 analytical column packed with 5 μm spherical particles.

Prostaglandins eluted from the HPLC-system were detected by adsorption in ultra violet. Underivated prostaglandins were detected at 199.5 nm. The fractions alpha-4, alpha-5 and alpha-6 evaporated to dryness and taken from Cowper's glands and Bartholini's glands respectively from dry column chromatography were each dissolved in 2 ml of absolute ethanol. 20 μl were taken out for each of these 2 ml samples. Of the Cowper's and Bartholini's glands, five such 20 μl samples were treated in the column for each fraction alpha-4, alpha-5, and alpha-6. A three-component eluant comprising phosphoric acid (0.1 M), acetonitrile, methanol in ratios 40:12:48 was used to separate the fractions on the column. Samples were then taken systematically from the column and analysed with respect to the contraction of gerbil colon. The fractions with retention times (RT) 3—6 and 12—15 from fraction alpha-4, the fractions with retention time 3—6 and 12—15 from alpha-5 and those with retention time 8—10 from alpha-6 were observed to contract gerbil colon. This applies to alpha-4, alpha-6 and alpha-6 from both Cowper's and Bartholini's glands.

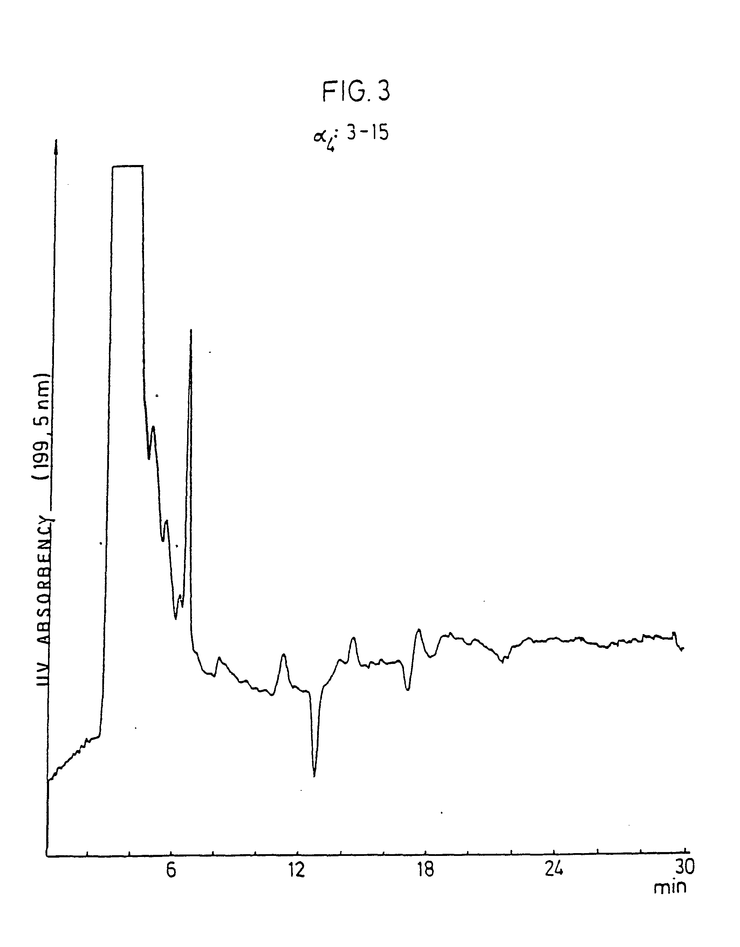

When compared with a number of prostaglandin standards, such as $PGE_2$, $PGI_2$ and $PGE_1$, and also $PGF_{2a}$ and $PGA_2$, none of these prostaglandins could be observed in the mixture (c.f. Fig. 2, which illustrates the UV-adsorption at 199.5 nm as a function of eluting volume and retention time at HPLC of said standards, with Figs. 3, 4 and 5, which illustrate the same curve for the fractions $a_4$, $a_5$, $a_6$ from DCC). The biological activity was found to be complex, however, when fractionation was effected with HPLC. The primary biological activity was found in HPLC-fractions alpha-4:12—15, alpha-5, 12—15 and alpha-6: 8—10.

Gas chromatographic-mass spectrometric analysis

This analysis showed a complex mixture where, however, certain fragments were observed which indicated the presence of neurotransmitter-like substances having a molecular weight of between 200—1000 m/c (alpha-6: 8—10).

NMR-analysis

NMR (nuclear magnetic resonance) was used to investigate the fraction alpha-5: 12—15, a fraction of high biological activity—it exhibited all characteristics of prostaglandin-like substances, such as long chain, the presence of fatty acids, terminal $CH_3$-group.

Example 2

50 g of Cowper's glands taken from deceased men were homogenized with 50 ml methanol in Ultraturrax. The pH was adjusted to 4.5 with 0.1 M potassium hydrogen phosphate. The extract was allowed to stand overnight in a freezer, whereafter ice crystals were filtered-off at a temperature beneath 0°C. The sample was then concentrated by evaporating to dryness in a nitrogen atmosphere in a water bath.

A number of experiments are given below which illustrate the biological effect of the factor according to the invention.

Experiment 1

The effect of the factor on muscle-collagen-trabecules in corpora cavernosa of humans and mammals (bull) the effect on the smooth muscle of the penis arteries.

The erection of the penis of the human male and male mammals is due to an overfilling of the erectile tissues in the penis, i.e. corpora cavernosa (swelling bodies). These contain a sponge-like tissue comprising collagent tissue bundles and smooth muscle filaments which form an extensive network, the so-called trabecula corpora cavernosa. The cavities formed can be rapidly expanded and contracted, i.e. filled and emptied of blood. With an erection, the smooth musculature in corpora cavernosa relaxes. In addition, the arteries supplying corpora cavernosa with blood, i.e. the penis arteries, are dilated. Erection of the penis is terminated, i.e. the penis relaxes, in a similar manner, by corresponding contraction of the muscle filaments in corpora cavernosa and the penis arteries.

Preparation

A number of male animals were slaughtered and the whole penis of each removed and transported to the laboratory in a buffer solution (Krebs solution) at a temperature of +4°C. Parts of the corpora cavernosa of rams were removed in a similar fashion. Long strips measuring ca 1.5 cm in length and ca 4 cm in width were cut from the centre part of the musculature in the trabeculae network. Similarly, a strip having the length of 1.5 cm and a width of ca 3 mm was cut from the musculature of the penis artery. The dissection was complected with 1—2 h after slaughtering the animal. The strips were kept in the Krebs-solution for a maximum of one calendar day, and exhibited no loss in activity during this period.

Bioanalysis: The effect of the factor on the isolated muscle filaments was studied with the aid of a Grass Force Deplacement Transducer coupled to a Grass-polygraph. $PGE_2$ and $PFG_{2\alpha}$ were used as standards during this study. By avoiding excessive tension in the fastening means of the bio assay system, it was possible to retain spontaneous motility in the tissue. The factor deriving from Cowper's glands was dripped onto the isolated muscle fibres, wherewith there was obtained a clear relaxation of the muscles at the trabeculae network from the penis. The same reaction—although weaker—was obtained with the fraction deriving from Bartholini's glands. $PGE_2$ in a concentration of 50 ng/ml gave a similar effect, although extremely weak, while $PGF_{2\alpha}$ in corresponding concentration gave instead an increased muscle-tonus.

The factor from Cowper's glands was dripped onto the penis artery suspended in the bioanalysis system in a manner similar to the aforesaid muscle fibres. A clear relaxation of the musculature was observed, when the factor dripped onto the penis artery. On the other hand, $PGF_{2\alpha}$, concentration 50 ng/ml and $PGE_2$ in the same concentration, resulted in strong contraction of the penis artery. This appears to show clearly that while the two types of prostaglandin used primarily counteract erection, the lipid fractions deriving from the glands strongly promote erection.

Experiment 2

The factor deriving from the glands contracts the urethra musculature of male humans and male mammals. The urethra comprises a mucous-membrane duct, the majority of which is enclosed by a layer of muscle with internal, longitudinally extending musculature and outer transverse layers. It is best developed in the rear part of the urethra. In the forward part of the urethra there are found slanting bundles of muscle. When operating on the prostate of the male genital system, there is removed from the urethra within the prostate section a part of the urethra, which is prepared and stored in a Krebs-buffer. In the bioanalysis test, the prepared strips of muscle were mounted in the aforedescribed bioanalysis assembly, with extreme sensitivity. A mixture of $O_2$ (95%) and $CO_2$ (5%) at 37°C was added to the organ bath. The factor deriving from Cowper's glands was dripped onto the muscle strips, whereupon a pronounced contraction of the urethra musculature was observed. The same effect, although less pronounced, was obtained with the factor deriving from Bartholini's glands. Both of the factors were extracted from the glands of human beings. The results have since been verified with urethra from the bull; $PGF_{2\alpha}$ resulted in similar contraction of urethra musculature while $PGE_2$ relaxed the musculature. The contraction of urethra musculature when exposed to said factor must promote the ejaculation process. It is true that the method of measuring this function is indirect, but at the present time there are no methods whereby the function can be measured directly on human beings.

Experiment 3

The factor promotes motility of the spermatozoa. It is known that E-type prostaglandins have no effect on spermatozoon mobility. Prostaglandin $F_{2\alpha}$ possibly has a retarding effect on spermatozoon motility. In tests carried out by us on the factor, the motility of spermatozoon was studied under a microscope. In so doing, Bartholini's glands and Cowper's glands respectively were dissolved in saline and added to a solution containing active spermatozoa.

It could be shown that the factor deriving from Bartholini's glands created a particularly active potentiation of the mobility of the spermatozoa. The factor deriving from Cowper's glands gave a similar, although less pronounced effect at the same concentration. The saline had no effect. The spermatozoa, from humans, sheep, and bulls, are emptied upon ejaculation into the vagina and are directed to reach the ovum by their own mobility. Thus, there are good grounds to suppose that the factor facilitates active migration of the spermatozoa through the vagina and the uterine cavity.

Experiment 4

The factor promotes penetration of spermatozoa through fresh egg white. A hens egg was crushed and the egg white placed on a microscope slide. A capillary tube was then introduced into the egg white and the egg white withdrawn. A spermatozoon sample was placed on each of two watch glasses, one with the factor applied thereto and the other with no factor. The factor deriving from both the Bartholini's glands and the Cowper's glands, from human beings, were used in respective test procedures. The tubes containing egg white were placed in the spermatozoon samples and fixated with plasticine (modelling clay). The capillaries were positioned vertically and the watch glasses, with capillaries placed thereon, wers then placed in a heating cabinet. The ability of the spermatozoa to migrate through the egg white was then studied under a microscope at time intervals of 60, 120 and 180 minutes. It was clearly seen that the factor deriving from the Bartholini's glands significantly increased the ability of the spermatozoa to pass through the egg white; the factor deriving from the Cowper's glands had a similar, although weaker effect.

The ability of the spermatozoa, to penetrate the protein in the ovum cells is of the greatest significance with respect to fertilization.

Experiment 5

The factor coagulates semen (the fluid in which spermatozoa are suspended). Fibrinolytic enzymes in semen provide complete or partial liquefaction of coagulated spermatozoa. It is known that total extract of the secretion from the Cowper's glands derived from bulls and from rams coagulates semen. Expressed functionally, liquid semen forms a coagulation clot, which constitutes an important feature in the ejaculation process. It also prevents reflux of semen during the ejaculation phase and thus promotes the fertility of animals, and is particularly promotive in rams.

The ability of the factor to coagulate spermatozoa is illustrated by the following: 0.5 ml of liquefied spermatozoa (in a readily flowable state) was introduced into a test tube at 37°C. 0.5 ml of the factor (derived from Cowper's glands) was dissolved in saline and added to the liquid spermatozoa. The spermatozoa were found to coagulate rapidly, to form a solid coagulate. This coagulate was seen to return to liquid form after about 10 minutes. Addition of the factor derived from the Bartholini's glands gave a similar, although weaker coagulation.

Experiment 6

The factor contracts smooth musculature in the uterotubal junction of women (UTJ). When the factor is placed on smooth musculature from UTJ, a composite response is obtained. This is because the female uterotubal junction contains three different muscle layers which give specific response to lipid mediators within the prostaglandin family. Muscle tissue was isolated from UTJ taken from healthy fertile women who had undergone sterilization or an hysterotomy. Circular and longitudinal muscle filaments were introduced into a bioanalysis unit with a Krebs bicarbonate buffer. As is normal, a mixture of oxygen and carbon dioxide was added, and the tests were carried out at a temperature of 37°C. The factor was found to create contraction of the longitudinal musculature and relaxation in the circular musculature.

Experiment 7

The factor contracts vaginal musculature. Smooth musculature isolated from the vaginal walls of cows was arranged in the bioanalysis unit. The factor derived from Cowper's glands extracted from bulls produced a marked contraction of strips of muscle. It is well known that the penis arteries are dilated with erection, more blood is supplied to the corpora cavernosa, which is filled as a result thereof and an erection takes place.

In the process of ejaculation the musculature around the urethra is drawn together in a plurality of autonomous contractions, and ejects the semen from the penis at high pressure. The spermatozoa present in the semen ejaculated are deposited in the vagina and move, either through active mobility or passive migration, to the uterine coverty, from whence the spermatozoa migrate further and reach the ovum, which is subsequently penetrated.

Experiment 8—Erection test

Monkeys (guenons) from 3 to 4 years age and weighing 4.5—5.0 kg were put to sleep with ketalar 1.8 ml+optionally a further 0.5 ml. The penis of respective monkey was held retarded with the aid of an elongated suction device.

The following substances were injected through tunica albuginea in corpus cavernosum (smooth musculature) with the aid of a syringe 26 G point, the solution injected had a temperature of 30°C.

1. 0.5 ml physiological sodium chloride (NaCl), which gave no reaction;

2. 0.5 ml (15 mg) papaverine, which promoted an erection of a few minutes; the erection remained 1—2 hours;

3. 0.5 ml (2 mg) phenoxy benzamine, which gave the same reaction as papaverine, possibly of longer duration;

4. A freeze-dried substance obtained in Example 2 was dissolved in 0.5 ml physiological NaCl after blowing in nitrogen. Three monkeys were tested. Erection was observed and lasted for 2—4 hours for 2 animals and up to 24 hours for 1 animal.

It has been possible to show that the factor derived from the glands of human beings and mammals (Cowpwer and Bartholini glands) clearly promote both erection and ejaculation, and fertilize the ovum by:

—dilating the musculature incorpora cavernosa in penis;

—dilating the musculature of the penis artery;

—contracting the urethra musculature;

—contracting the vaginal musculature;

—contracting the longitudinal bundles of the uterotubal junction (uterus-oviduct juncture);

—potentiating the active mobility of spermatozoa;

—increasing the ability of spermatozoa to penetrate albumen (protein); and

—coagulating semen.

When taken together with the known fact that extract from the glands serves to lubricate urogenital mucous membrane, there is obtained hereby an indication that the factor has a true significance in the promotion of erection, ejaculation and the mobility of spermatozoa and its ability to penetrate protein. The factor can thus be used as a fertility promoting substance.

The manner in which the factor exerts its effect in an *in vivo* situation can be illustrated by taking a starting point from our pioneer work with electron-microscope studies on the glands, primarily Cowper's glands (L. Hellgren, E. Mylius and J. Vincent: The ultra-structure of the human bulbo-urethral gland. J. Submicrosc. Cytol. 14 (4), 683—689, 1982). The factor can be conceived to have either a direct or an indirect effect, or a combination of the two. The direct effect can be conceived to act on the spermatozoa in accordance with experiments 1—7. The indirect ("hormonal") effect of the factor on the musculature of the penis artery, the vagina, urethra and oviduct or Fallopian tubes may be achieved through blood circulation. That this is probably so is supported by our electron-microscopic discovery of secretory granules in Cowper's and Bartholini's glands present in the gland blood vessels, the granular content probably being emptied directly into the circulation.

The factor can be used as a fertility and erection promoting agent. These agents may constitute such lipid fractions from Cowper's and Bartholini's glands as those which contract gerbil colon, e.g. the first impure total lipid extract, and in particular the active fractions, eluted in dry column chromatography on high pressure liquid chromatography in Example I. The agents may also be present in compositions together with conventional extenders. The compositions may be in the form of injectable liquids, sprays, tablets, suspensions, solutions, syrups, suppositories and capsules. The sample solutions are preferably evaporated to dryness and dissolved in physiological cooking salt and injected.

## Claims

1. An erection and fertility promoting agent, characterized in that it contains as an active component such lipid fractions from Cowper's and/or Bartholini's glands taken from human beings and/or mammals as those which contract gerbil colon.

2. A method of producing an erection and fertility promoting agent according to Claim 1, characterized by optionally first freeing the glands from proteins, extracting the glands with a lipid solvent in an acid environment, and removing water and optionally evaporating off the solvent, and purifying the residue by chromatography and isolating such fractions as those which contract gerbil colon, whereafter said fractions are optionally further purified by chromatography, preferably high pressure liquid chromatography.

3. A method according to Claim 2, characterized by extracting the glands with a lipid solvent in the form of ether, ethyl acetate, isopropanol, methanol, acetone, chloroform, water and mixtures thereof, optionally in a multiple of stages, preferably with methanol or ethyl acetate and mixtures thereof, in particular with ethyl acetate and methanol 3/1 vol/vol.

4. A method according to Claim 2, characterized in that the chromatography process is effected on silica gel while eluting with a mixture of ethyl acetate, isooctane, ethanol, acetic acid, water, chloroform, isopropanol, formic acid, preferably a mixture of ethyl acetate, isooctane, ethanol acetic acid and water (at ratios of 35:10:3:0.1:0.1) vol/vol, or chloroform, isopropanol, ethanol, formic acid at ratios of 45:5:0.5:0.3 vol/vol.

5. A method according to Claim 2, characterized in that said further purification of said such fractions is effected by high pressure liquid chromatography while eluting with mixtures of phosphoric acid, acetonitrile, methanol, preferably in ratios of 40:12:48 vol/vol.

6. The use of an agent produced in accordance with any one of Claims 2—5 for preparing an erection or fertility promoting agent.

7. The use according to Claim 6 for producing an erection and fertility promoting agent in the form of compositions having the form of injectionable liquids, sprays, tablets, suspensions, solutions, syrups, suppositories or capsules, containing conventional additives and extenders.

## Patentansprüche

1. Ein die Erektion und Fruchtbarkeit fördernder Wirkstoff, dadurch gekennzeichnet, daß er als eine aktive Komponente solche Lipoidfraktionen von Cowperschen und/oder Bartholinischen Drüsen, die von Menschen und/oder Säugetieren entnommen sind, enthält, die Wüstenspringmausdickdarm zusammenziehen.

2. Verfahren zum Herstellen eines die Erektion und Fruchtbarkeit fördernden Wirkstoffs nach Anspruch 1, dadurch gekennzeichnet, daß ggf. zunächst die Drüsen von Proteinen befreit werden, die Drüsen mit einem Lipoidlösungsmittel in einer sauren Umgebung extrahiert werden und Wasser entfernt und ggf. das Lösungsmittel verdampft wird und der Rückstand durch Chromatographie gereinigt und solche Fraktionen isoliert werden, die Wüstenspringmausdickdarm zusammenziehen, wonach die Fraktionen ggf. durch Chromatographie weiter gereinigt werden, vorzugsweise durch Hochdruckflüssigkeitschromatographie.

3. Verfahren nach Anspruch 2, gekennzeichnet durch Extraktion der Drüsen mit einem Lipidlösungsmittel in Form von Äther, Äthylacetat, Isopropanol, Methanol, Aceton, Chloroform, Wasser und Mischungen daraus, ggf. in mehreren Stufen, vorzugsweise mit Methanol oder Äthylacetat und Mischungen davon, und speziell mit Äthylacetat und Methanol 3/1 Vol/Vol.

4. Verfahren nach Anspruch 2, dadurch gekennzeichnet, daß der Chromatographieprozeß auf Silicagel ausgeführt wird, mit Eludierung mit einem Gemisch von Äthylacetat, Isooctan, Äthanol, Essigsäure, Wasser, Chloroform, Isopropanol, Ameisensäure, vorzugsweise einem Gemisch von Äthylacetat, Isooctan, Äthanol, Essigsäure und Wasser (bei Verhältnissen von 35:10:3:0,1:0,1) Vol/Vol, oder Chloroform, Isopropanol, Äthanol, Ameisensäure bei Verhältnissen von 45:5:0,5:0,3 Vol/Vol.

5. Verfahren nach Anspruch 2, dadurch gekennzeichnet, daß die weitere Reinigung der genannten Fraktionen durch Hochdruckflüssigkeitschromatographie ausgeführt wird mit Eludierung mit Mischungen aus Phosphorsäure, Acetonitril, Methanol, vorzugsweise in Verhältnissen von 40:12:48 Vol/Vol.

6. Verwendung eines Wirkstoffs, der gemäß einem der Ansprüche 2 bis 5 hergestellt ist, zur Herstellung eines die Erektion oder Fruchtbarkeit begünstigenden Wirkstoffs.

7. Verwendung nach Anspruch 6 zur Erzeugung eines die Erektion und Fruchtbarkeit begünstigenden Wirkstoffs in Form von Zusammensetzungen, die die Form von injizierbaren Flüssigkeiten, Sprays, Tabletten, Suspensionen, Lösungen, Sirupen, Zäpfchen oder Kapseln haben und die übliche Zusätze und Streckmittel enthalten.

## Revendications

1. Agent stimulant l'érection et la fertilité, caractérisé en ce qu'il contient un principe actif tel que les fractions lipidiques provenant de glandes de Cowper et/ou de Bartholini prélevées sur des êtres humains et/ou sur des mammifères ainsi que celle squi contractent le côlon de la gerbille.

2. Méthode de fabrication d'un agent stimulant l'érection et la fertilité selon la revendication 1, caractérisée en ce que les protéines sont éventuellement préalablement éliminées des glandes, en soumettant les glandes à une extraction au moyen d'un solvant des lipides en milieu acide, en éliminant l'eau at en évaporant éventuellement le solvant et en purifiant le résidu par chromatographie et en isolant les fractions qui contractent le côlon de la gerbille, après quoi lea dites fractions subissent éventuellement une purification ultérieure par chromatographie, de préférence par chromatographie liquide sous haute pression.

3. Méthode selon la revendication 2, caractérisée en ce que l'on extrait les glandes au moyen d'un solvant des lipides tel que de l'éther, de l'acétate d'éthyle, de l'isopropanol, du méthanol, de l'acétone, du chloroforme, de l'eau et leurs mélanges, éventuellement en plusieurs étapes, de préférence au moyen de méthanol ou d'acétate d'ethyle et leurs mélanges, en particulier au moyen d'un mélange d'acétate d'éthyle et de méthanol dans un rapport en volume de 3/1.

4. Méthode selon la revendication 2, caractérisée en ce que le procédé chromatographique se pratique sur gel de silice en éluant au moyen d'un mélange d'acétate d'éthyle, d'iso-octane, d'éthanol, d'acide acétique, d'eau, de chloroforme, d'isopropanol, d'acide formique, de préférence un mélange d'acétate d'éthyle, d'iso-octane, d'éthanol, d'acide acétique et d'eau (dans les rapports en volume de 35/10/3/0,1/0,1) ou de chloroforme, d'isopropanol, d'éthanol, d'acide formique dans les rapports en volume de 45/5/0,5/0,3.

5. Méthode selon la revendication 2, caractérisée en ce que la dite purification ultérieure des dites fractions s'effectue par chromatographie en phase liquide sous haute pression en éluant au moyen de mélanges d'acide phosphorique, d'acétonitrile, de méthanol, de préférence dans les rapports en volume de 40/12/48.

6. L'utilisation de l'agent fabriqué selon l'une des revendications 2 à 5 à préparation d'un agent stimulant l'érection et la fertilité.

7. L'utilisation selon la revendication 6 pour la fabrication d'un agent stimulant l'érection et la fertilité sous forme de préparations ayant la forme de liquides injectables, sprays, comprimés, suspensions, solutions, sirops, suppositoires ou gélules, contenant des additifs et diluants classiques.

# FIG.1

ISOLATION

$\downarrow$

EXTRACTION

$\downarrow$

DRY COLUMN CHROMATOGRAPHY
$\left\{\begin{array}{l} \alpha_1 \\ \alpha_2 \\ \vdots \\ \alpha_{10} \end{array}\right.$

$\downarrow$

BIOANALYSIS $\quad \alpha_4, \alpha_5$ AND $\alpha_6$ ACTIVE

$\downarrow$

HIGH PRESSURE LIQUID CHROMATOGRAPHY

$\alpha_4 : 3-15$ FIG. 3

$\alpha_5 : 3-15$ FIG. 4

$\alpha_6 : 3-15$ FIG. 5

$\downarrow$

BIOANALYSIS $\quad \alpha_4 : 12-15, \alpha_5 : 12-15$ AND $\alpha_6 : 8-10$ ACTIVE

EP 0 172 873 B1

EP 0 172 873 B1

FIG.2

PROSTAGLANDIN STANDARDS

2

FIG. 3

$\alpha_4$: 3-15

$\alpha_5 : 3\text{-}15$

FIG. 5
$\alpha_6$ : 3-15